# EUROPÄISCHE PATENTSCHRIFT

(11) **EP 2 337 534 B1**
(45) Veröffentlichungstag und Bekanntmachung des Hinweises auf die Patenterteilung: **06.04.2016**
(21) Anmeldenummer: 08773773.0
(22) Anmeldetag: 30.06.2008
(51) Int. Cl.: A61F 9/01, A61B 3/10, A61B 3/113, A61B 3/117, A61F 9/008

(54) **VORRICHTUNG, VERFAHREN UND STEUERPROGRAMM FÜR OPHTHALMOLOGISCHE, INSBESONDERE REFRAKTIVE LASERCHIRURGIE**
DEVICE, METHOD AND CONTROL PROGRAM FOR OPHTHALMOLOGIC, PARTICULARLY REFRACTIVE, LASER SURGERY
DISPOSITIF, PROCÉDÉ ET PROGRAMME DE COMMANDE DE CHIRURGIE LASER, NOTAMMENT RÉFRACTIVE, EN OPHTALMOLOGIE

(43) Veröffentlichungstag der Anmeldung: 29.06.2011
(73) Patentinhaber: WaveLight GmbH, 91058 Erlangen (DE)
(72) Erfinder: RIEDEL, Peter, 90489 Nürnberg (DE); DONITZKY, Christof, 90542 Eckental (DE)
(74) Vertreter: Katérle, Axel
(86) Internationale Anmeldenummer: PCT/EP2008/005332
(87) Internationale Veröffentlichungsnummer: WO 2010/000278

(56) Entgegenhaltungen:
- WO-A-2007/143111
- US-A1- 2004 143 246
- US-B1- 6 726 680

## Beschreibung

Die Erfindung betrifft eine Vorrichtung für die ophthalmologische, insbesondere refraktive Laserchirurgie. Sie betrifft ferner ein Steuerprogramm für eine solche Vorrichtung sowie ein Verfahren zum Erzeugen eines solchen Steuerprogramms.

Unter refraktiver Laserchirurgie sei hier die Änderung der Abbildungseigenschaften des optischen Systems "Auge" mittels Laserstrahlung verstanden. Die Wechselwirkung der eingestrahlten Laserstrahlung mit dem Auge ändert die Brechungseigenschaften einer oder mehrerer Komponenten des Auges. Da für die Abbildungseigenschaften des Auges vor allem die Hornhaut (Kornea) maßgeblich ist, beinhaltet die refraktive Laserchirurgie des Auges in vielen Fällen eine Bearbeitung der Kornea. Durch gezieltes Einbringen von Schnitten oder/und gezielten Materialabtrag wird dabei eine Formänderung der Kornea bewirkt; man spricht deshalb auch von einer Neuformung.

Prominentes Beispiel einer Neuformung der Kornea zur Änderung ihrer refraktiven Eigenschaften ist die LASIK (Laser-In-Situ-Keratomileusis). Bei der LASIK wird zunächst aus der Kornea ein oberflächliches Deckelscheibchen herausgeschnitten, das in der Fachwelt gemeinhin als Flap bezeichnet wird. Der Flap hängt an einem Teil seines Rands noch an dem danebenliegenden Hornhautgewebe, so dass er problemlos zur Seite geklappt und später wieder zurückgeklappt werden kann. Zur Erzeugung des Flaps kommen in der bisherigen Praxis insbesondere zwei Methoden zum Einsatz, zum einen eine mechanische mittels eines Mikrokeratoms und zum anderen eine lasertechnische, bei der mittels Femtosekunden-Laserstrahlung (d.h. gepulster Laserstrahlung mit einer Pulsdauer im fs-Bereich) ein flächiger Tiefenschnitt in die Kornea eingebracht wird, der bis auf den Gelenkbereich zur Korneaoberfläche herausgeführt wird. Nach Wegklappen des erzeugten Flaps erfolgt ein Materialabtrag (Ablation) vom so freigelegten Stroma gemäß einem vorbestimmten Ablationsprofil. Das Ablationsprofil gibt an, an welcher Stelle der Kornea wieviel Gewebe abzutragen ist. Das Ablationsprofil wird so berechnet, dass nach der Ablation die Kornea eine für das behandelte Auge optimale Form hat und die zuvor vorhandenen optischen Abbildungsfehler des Auges weitestmöglich korrigiert sind. Für die Berechnung des Ablationsprofils stehen der Fachwelt seit längerem geeignete Methoden zur Verfügung.

Für die Ablation kommt beispielsweise ein Excimer-Laser mit einer Strahlungswellenlänge im UV-Bereich zum Einsatz, etwa bei 193 nm.

Ist für das zu behandelnde Auge das Ablationsprofil bestimmt, wird anschließend berechnet, wie der gewünschte Abtrag mit der zur Verfügung stehenden Laserstrahlung am besten erzielt werden kann. Die verwendete Laserstrahlung ist regelmäßig gepulste Strahlung. Es geht deshalb darum, eine Folge von Laserpulsen nach Raum und Zeit zu berechnen, die in Wechselwirkung mit der Kornea, insbesondere dem Stroma, die gewünschte Neuformung der Kornea bewirkt.

Strahlführungsmittel, um einen Laserstrahl so über das zu behandelnde Auge zu führen, dass sich die gewünschte Raum- und Zeitfolge von Laserpulsen einstellt, sind als solche im Stand der Technik bekannt. Insbesondere können die Strahlführungsmittel eine der Ablenkung des Laserstrahls in Querrichtung (x-y-Richtung) dienende Ablenkeinheit, bekannt auch als Scanner, sowie eine Fokussieroptik zur Fokussierung des Laserstrahls an einer gewünschten Höhenposition (z-Richtung) umfassen. Die Ablenkeinheit kann beispielsweise einen oder mehrere galvanometrisch gesteuerte Ablenkspiegel umfassen.

Die vorliegende Erfindung ist nicht auf die LASIK-Technik beschränkt. Sie kann auch bei anderen laserchirurgischen Eingriffen am Auge zum Einsatz kommen, etwa bei der PRK (Photorefaktive Keratektomie), LASEK, EPI-LASIK oder bei inzisionalen Verfahren, bei denen lediglich Schnitte in der Hornhaut gesetzt werden. Im Übrigen besteht auch keine Beschränkung der Erfindung auf eine laserchirurgische Behandlung der Kornea; auch für Behandlungen beispielsweise der Linse ist eine Anwendung der Erfindung vorstellbar.

Die erwähnten Strahlführungsmittel werden mittels eines programmgesteuerten Rechners nach Maßgabe des Ablationsprofils - oder allgemeiner nach Maßgabe eines Bearbeitungsprofils - gesteuert. Soweit es sich um eine nicht ablatierende chirurgische Behandlung handelt, kann das Bearbeitungsprofil beispielsweise auch ein Schnittprofil sein, das angibt, an welcher Stelle ein Schnitt wie tief zu setzen ist.

Das Bearbeitungsprofil benötigt einen Bezugspunkt (Referenzpunkt), auf den die Folge der Laserpunkte räumlich bezogen ist. Insbesondere im Zusammenhang mit der LASIK-Ablation ist vorgeschlagen worden, als Referenzpunkt für das Ablationsprofil den Mittelpunkt der Pupille zu verwenden. Die Pupille ist die von der Iris als Blende freigelassene Öffnung, durch welche hindurch Strahlung in das Auge und auf die Netzhaut gelangt; sie hat eine relativ scharfe Kontur und lässt sich deshalb gut mit einer Kamera aufnehmen und mit Bildverarbeitungsprogrammen auswerten. Geeignete Kameratechnik und Verarbeitungsprogramme sind im Stand der Technik verfügbar.

Das menschliche Auge ist jedoch kein ruhendes Objekt, sondern führt fortlaufend Bewegungen aus. Es gibt unterschiedliche Arten von Augenbewegungen, die zum Teil auf unterschiedlichen Zeitskalen ablaufen. Wichtig ist allein die Feststellung, dass das Auge niemals still steht. Dies gilt auch dann, wenn versucht wird, den Blick auf ein bestimmtes vorgegebenes Objekt zu fixieren; selbst dann treten unvermeidliche Fixationsbewegungen auf. Weil die Pupille die erwähnten Augenbewegungen in mehr oder weniger starkem Maß mitmacht, kann durch kameratechnische Beobachtung bzw. Überwachung der Pupille das Auge hinsichtlich seiner Bewegungen verfolgt werden. Entsprechende Augenverfolgungsgeräte (Eye-Tracker) verfolgen Bewegungen des Auges durch Aufnahme von Sequenzen von Bildern der Pupille einschließlich der umgebenden Iris und anschließende Auswertung der Bildsequenzen mittels Software. Im Rahmen der Bildauswertung wird insbesondere ermittelt, wo sich augenblicklich das Pupillenzentrum befindet. Indem das Ablationszentrum (Zentrum des Ablationsprofils) stets neu an dem so ermittelten Pupillenzentrum ausgerichtet wird, kann trotz der unvermeidlichen Augenbewegungen die gewünschte Raumfolge von Laserpulsen zuverlässig auf die richtigen Stellen des zu behandelnden Augenbereichs gelenkt werden.

Dokument US 2004/0143246 A1 beschreibt ein Verfahren, mit dem ein Laserstrahl genau auf eine gewünschte Position der Kornea ausgerichtet werden kann. Zur Messung einer Bewegung des Auges werden vier Marker verwendet. Alternativ kann die Bewegung durch Verzerrungen geometrischer Formen wie Ellipsen auf dem Auge detektiert werden. Aus der Berechnung der Augenbewegung kann bestimmt werden, ob das Pupillenzentrum auch eine von der Augenbewegung unabhängige Verschiebung erfährt, was bei der Positionierung des Laserstrahls entsprechend berücksichtigt wird.

Dokument US 6,726,680 B1 beschreibt ein Verfahren für Präzisionslaseranwendungen, das auf abgespeicherten Vorlagestrukturen für die Strahlführung basiert. Während einer Operation auftretende Augenbewegungen werden erkannt, indem die Lage bestimmter "Landmarks" auf der Kornea verfolgt wird. Dadurch können solche Bewegungen für eine stabilisierte Bildwiedergabe des zu behandelnden Auges kompensiert werden.

Dokument WO 2007/143111 A2 beschreibt ein Verfahren zur optischen Kohärenztomographie (OCT), bei dem Oberflächenbeschaffenheit und Dicke der Hornhaut bestimmt werden. Verfahren zur Bestimmung von Augenbewegungen während der Operation sind nicht näher beschrieben.

Die Verwendung des Pupillenzentrums als Referenzpunkt für das Ablationsprofil ist allerdings mit einem systematischen Nachteil verbunden, denn sie berücksichtigt nicht die Tiefe der vor der Pupille liegenden Augenvorderkammer und berücksichtigt auch nicht die Dicke der Kornea. Augenbewegungen sind regelmäßig Rotationsbewegungen, wobei der Drehpunkt im Innern des Glaskörpers liegt. Bewegt sich das Auge um einen bestimmten Winkel, so verschiebt sich das Pupillenzentrum in der Pupillenebene um einen ersten Wert, während sich ein an der Korneaoberfläche liegender Punkt in der Korneaebene um einen zweiten Wert verschiebt, der wegen des größeren Abstands des Korneapunkts vom Drehpunkt im Vergleich zum Abstand zwischen Pupillenzentrum und Drehpunkt größer als der erste Wert ist. Eine Ausrichtung des Ablationsprofils am Pupillenzentrum bringt deshalb bei Anwesenheit von Augenbewegungen Ungenauigkeiten mit sich.

Es kann deshalb daran gedacht werden, statt des Pupillenzentrums einen mit dem Pupillenzentrum in fester räumlicher Beziehung stehenden, patientenspezifischen Punkt an der Kornea als Bezugspunkt für das Ablationsprofil zu verwenden. Insbesondere kann dabei an den Durchstoßpunkt der Pupillenachse durch die Korneaoberfläche gedacht werden. Die Pupillenachse verläuft durch das Pupillenzentrum und durch die Korneaoberfläche. Bei Korneabehandlungen kann durch Verwendung eines an der Kornea liegenden Punkts als Referenzpunkt für das Bearbeitungsprofil der erwähnte systematische Fehler vermieden werden, der begangen wird, wenn stattdessen ein im Abstand von der Kornea liegender Punkt wie etwa das Pupillenzentrum als Bezugspunkt verwendet wird.

Für die geometrische Berechnung eines an der Kornea liegenden Bearbeitungszentrums aus dem Pupillenzentrum bedarf es Informationen über den Drehradius des Auges und den radialen Abstand zwischen den beiden Zentren. Letzterer wird hauptsächlich durch die Tiefe der Augenvorderkammer bestimmt; ein kleiner Teil dieses radialen Abstands wird ferner von der Hornhautdicke bestimmt.

Die Erfindung geht aus von der Erkenntnis, dass die Vorderkammertiefe einschließlich der Korneadicke bei unterschiedlichen Menschen in einem Maße variieren kann, dass es im Sinne einer Verbesserung des Operationsergebnisses günstig ist, konkret für den jeweiligen Patienten individuell dessen Vorderkammertiefe (gewünschtenfalls einschließlich der Korneadicke) messtechnisch zu ermitteln und diesen Messwert bei der Berechnung des kornealen Bearbeitungszentrums aus der Lage des Pupillenzentrums zu berücksichtigen. Beispielsweise konnte bei einer Versuchsgruppe von Patienten festgestellt werden, dass die Vorderkammertiefe einschließlich der Korneadicke innerhalb der Versuchsgruppe zwischen etwa 2,8 und 4,5 mm schwankte. Angesichts dieser festgestellten Schwankungsbreite ist es ein Aspekt der erfindungsgemäßen Lehre, dass die Annahme eines Standardwerts, beispielsweise 3,5 mm, für die Vorderkammertiefe inklusive der Korneadicke den tatsächlichen Verhältnissen bei einem aktuellen Patienten unter Umständen nur wenig gerecht wird und deswegen weiterhin von einem vergleichsweise starken Fehler ausgegangen werden muss, wenn das Bearbeitungszentrum unter Heranziehung eines solchen Standardwerts aus dem Pupillenzentrum berechnet wird.

Aufgabe der Erfindung ist es, für die ophthalmologische, insbesondere refraktive Laserchirurgie einen Weg aufzuzeigen, wie für ein vorgegebenes Bearbeitungsprofil während der Behandlung ein Bezugspunkt am Auge ermittelt werden kann, der verbesserte Operationsergebnisse erzielen lässt.

Erfindungsgemäß ist hierzu eine Vorrichtung für die ophthalmologische, insbesondere refraktive Laserchirurgie vorgesehen, umfassend
- eine Laserstrahlquelle,
- Strahlführungsmittel zum orts- und zeitgesteuerten Führen des von der Laserstrahlquelle emittierten Laserstrahls über ein zu behandelndes Auge,
- eine Kamera zum Aufnehmen eines Bilds der Iris und Pupille des Auges,
- einen mit der Kamera verbundenen, programmgesteuerten Rechner zum Steuern der Strahlführungsmittel nach Maßgabe eines Bearbeitungsprofils, wobei der Rechner dazu eingerichtet ist, während der Behandlung des Auges auf Grundlage der von der Kamera gelieferten Bilddaten die Lage eines vorgegebenen Punkts an der Hornhaut des Auges zu ermitteln und das Bearbeitungsprofil relativ zu der so ermittelten Lage des Hornhautpunkts auszurichten.

Erfindungsgemäß ist die Vorrichtung hierbei mit einer Messeinrichtung zur Messung eines für die Vorderkammertiefe und gewünschtenfalls die Hornhautdicke repräsentativen Tiefenmaßes des zu behandelnden Auges ausgestattet, wobei der Rechner mit den Messdaten der Messeinrichtung beliefert wird und dazu eingerichtet ist, die Lage des vorgegebenen Hornhautpunkts unter Berücksichtigung des gemessenen Tiefenmaßes zu ermitteln.

Die Erfindung lehrt somit, die Vorderkammertiefe und gewünschtenfalls die Hornhautdicke individuell für den jeweiligen Patienten zu vermessen und die Laserbearbeitung an einem Korneapunkt auszurichten, der unter Berücksichtigung dieser Messwerte ermittelt wurde. Vorzugsweise liegt der Bezugspunkt an der Vorderseite der Kornea. Die Messung kann unmittelbar vor Beginn der Chirurgie vorgenommen werden.

Seit einiger Zeit stehen kohärenzoptische interferometrische Messverfahren zur berührungslosen Vermessung biologischer Gewebe zur Verfügung, wie etwa die optische Kohärenztomographie (OCT) oder die Kohärenzbereichs-Reflektometrie (OLCR: Optical Low Coherence Reflectometry). Diese Messverfahren arbeiten mit breitbandiger Strahlung (z.B. SLED, ASE, Supercontinuum-Laser) und gestatten es, biologische Strukturen mit hoher Auflösung bis hin in den Bereich von 1 µm und feiner zu vermessen.

In einer bevorzugten Weiterbildung lehrt die Erfindung die Integration einer derartigen kohärenzoptischen interferometrischen Messeinrichtung in die laserchirurgische Vorrichtung, wobei es sich bei der Messeinrichtung insbesondere um eine OLCR-Messeinrichtung handelt. Die hohe Messgenauigkeit einer solchen Messeinrichtung erlaubt es, die Schwankungen der Vorderkammertiefe und der Hornhautdicke zwischen unterschiedlichen Patienten aufzulösen und präzise zu erfassen. Die Integration der Messeinrichtung in die laserchirurgische Vorrichtung ist insbesondere derart, dass der von der Messeinrichtung ausgesendete Messstrahl gleichachsig zu dem der Behandlung dienenden Laserstrahl auf das Auge gerichtet wird, so dass der Patient nur einmal positioniert werden muss und die Messung gegebenenfalls während der Operation wiederholt werden kann.

Nicht nur die Vorderkammertiefe kann von Mensch zu Mensch unterschiedlich sein sondern auch der Augendurchmesser insgesamt. Ein unterschiedlicher Augendurchmesser führt zu einem entsprechend unterschiedlichen Drehradius bei Rotationsbewegungen des Auges. Dementsprechend ist bei einer bevorzugten Ausführungsform vorgesehen, dass der Rechner dazu eingerichtet ist, die Lage des vorgegebenen Hornhautpunkts auch unter Berücksichtigung eines patientenspezifischen, präoperativ ermittelten Drehradius des Auges zu ermitteln. Die Berücksichtigung des individuell gemessenen Drehradius gestattet weitere Verbesserungen im Vergleich zur Verwendung eines standardmäßig vorgegebenen Drehradius.

Wie bereits erwähnt, kann als vorgegebener Hornhautpunkt der Durchstoßpunkt der Pupillenachse durch die Hornhautoberfläche verwendet werden. Alternativ kann ein mit diesem Durchstoßpunkt in fester relativer Lage stehender Hornhautpunkt verwendet werden.

Insbesondere bei LASIK-Eingriffen, wo zunächst der Flap weggeklappt wird, um anschließend die Ablation durchzuführen, besteht während der Ablation keine Möglichkeit, die Hornhautoberfläche unmittelbar nach einem vorgegebenen Punkt abzusuchen. Der Referenzpunkt für das Ablationszentrum kann dementsprechend nur auf mittelbarem Wege berechnet werden. Eine Möglichkeit hierzu kann darin bestehen, während der Laserbehandlung aus den Bilddaten der Kamera fortlaufend ein aktuelles Verschiebungsmaß für das Pupillenzentrum zu ermitteln, das die Verschiebung der aktuellen Lage des Pupillenzentrums gegenüber einer gegebenen Referenzlage angibt. Das Verschiebungsmaß des Pupillenzentrums kann insbesondere in Form eines Verschiebungsvektors bestimmt werden, der die Richtung und Stärke der Verschiebung des Pupillenzentrums gegenüber der Referenzlage repräsentiert. Augenbewegungen, die im Verlauf des Lasereingriffs erfolgen, können dann jeweils durch einen auf diese Referenzlage des Pupillenzentrums bezogenen Verschiebungsvektor ausgedrückt werden.

Unter Verwendung des gemessenen Tiefenmaßes kann sodann ein dem Verschiebungsmaß des Pupillenzentrums entsprechendes Verschiebungsmaß für den vorgegebenen Hornhautpunkt berechnet werden, beispielsweise wiederum in Form eines Verschiebungsvektors. Die aktuelle Lage des vorgegebenen Hornhautpunkts kann aus dem so berechneten Verschiebungsmaß des vorgegebenen Hornhautpunkts und einer bekannten Referenzlage für diesen Punkt ermittelt werden. Die Referenzlage des vorgegebenen Hornhautpunkts ist dabei zweckmäßigerweise diejenige Lage, die der vorgegebene Hornhautpunkt einnimmt, wenn sich das Pupillenzentrum in dessen Referenzlage befindet. Beispielsweise können am Anfang der Operation einmal die Lage des Durchstoßpunkts der Pupillenachse durch die Korneaoberfläche und die zugehörige Lage des Pupillenzentrums ermittelt und als Referenzlagen für den vorgegebenen Hornhautpunkt und das Pupillenzentrum gespeichert werden.

Die Erfindung stellt ferner ein Steuerprogramm für eine Vorrichtung für die ophthalmologische, insbesondere refraktive Laserchirurgie bereit, wobei die Vorrichtung eine Laserstrahlquelle, Strahlführungsmittel zum orts- und zeitgesteuerten Führen des von der Laserstrahlquelle emittierten Laserstrahls über ein zu behandelndes Auge, eine Kamera zum Aufnehmen eines Bilds der Iris und Pupille des Auges und einen mit der Kamera verbundenen, das Steuerprogramm ausführenden Rechner zum Steuern der Strahlführungsmittel nach Maßgabe eines Bearbeitungsprofils umfasst. Das Steuerprogramm ist derart ausgestaltet, dass der Rechner während der Behandlung des Auges auf Grundlage der von der Kamera gelieferten Bilddaten die Lage eines vorgegebenen Punkts an der Hornhaut des Auges ermittelt und das Bearbeitungsprofil relativ zu der so ermittelten Lage des Hornhautpunkts ausrichtet. Das Steuerprogramm ermittelt dabei die Lage des vorgegebenen Hornhautpunkts unter Berücksichtigung eines für die Vorderkammertiefe und gewünschtenfalls die Hornhautdicke repräsentativen gemessenen Tiefenmaßes des zu behandelnden Auges.

Das Steuerprogramm kann beispielsweise auf einem maschinenlesbaren transportablen Datenträger abgelegt oder in einem Speicherbaustein gespeichert sein, auf den der Rechner zugreifen kann.

Des weiteren betrifft die Erfindung ein Verfahren zum Erzeugen eines Steuerprogramms für einen programmgesteuerten Rechner einer Vorrichtung für die ophthalmologische, insbesondere refraktive Laserchirurgie, wobei die Vorrichtung dazu eingerichtet ist, Laserstrahlung gemäß einer durch ein gewünschtes Bearbeitungsprofil bestimmten, relativ zu einem vorgegebenen Ort eines zu behandelnden Auges ausgerichteten Raum- und Zeitfolge auf oder in das Auge zu richten. Erfindungsgemäß ist bei diesem Verfahren vorgesehen, dass zumindest einmal vor der Chirurgie ein für die Vorderkammertiefe und gewünschtenfalls die Hornhautdicke repräsentatives Tiefenmaß des zu behandelnden Auges gemessen wird und das Steuerprogramm so erzeugt wird, dass es während der Chirurgie den vorgegebenen Wert des Auges unter Berücksichtigung des gemessenen Tiefenmaßes ermittelt.

Für das Steuerprogramm und das Verfahren gelten die zuvor im Zusammenhang mit der erfindungsgemäßen laserchirurgischen Vorrichtung gemachten Anmerkungen und erläuterten bevorzugten Ausführungsbeispiele in entsprechender Weise.

Die Erfindung wird nachfolgend anhand der beigefügten Zeichnungen weiter erläutert. Es stellen dar:
Figur 1 in schematischer Blockdarstellung ein Ausführungsbeispiel einer Vorrichtung für die refraktive Laserchirurgie des Auges,
Figur 2 eine Schnittdarstellung des vorderen Augenbereichs und
Figur 3 eine Schnittdarstellung des vorderen Augenbereichs in einer gegenüber Figur 2 verdrehten Stellung des Auges.

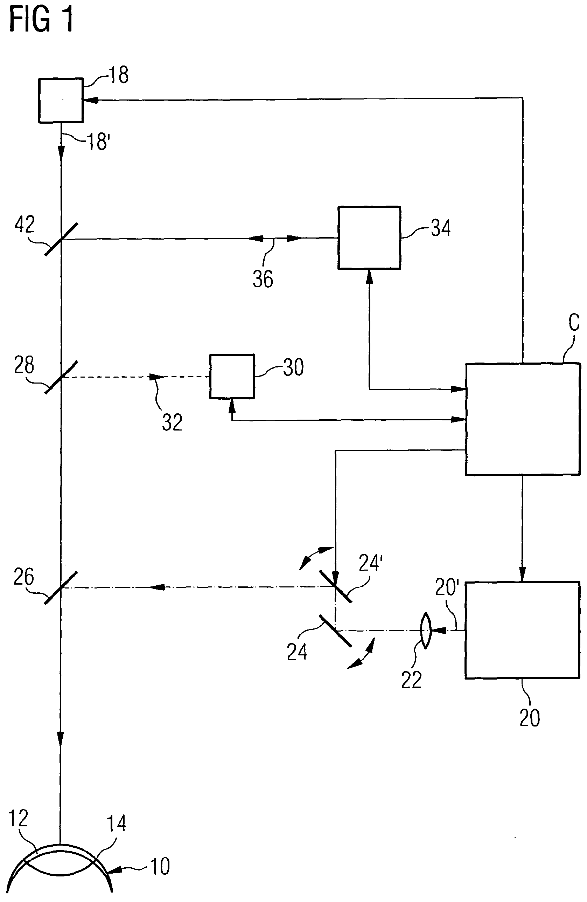

In Figur 1 ist ein mit refraktiver Laserchirurgie zu behandelndes Auge schematisch bei 10 angedeutet. Die Kornea des Auges 10 sowie der Pupillenrand sind bei 12 bzw. 14 gezeigt.

Die laserchirurgische Vorrichtung gemäß Figur 1 weist in an sich bekannter Weise eine Fixationslichtquelle (z.B. LED oder Laser) 18 auf, die einen (schwachen) Fixationsstrahl 18' emittiert und vom Patienten zur Fixierung des Auges anvisiert wird.

Die laserchirurgische Vorrichtung umfasst ferner einen Behandlungslaser 20, der Behandlungsstrahlung 20' emittiert, die über eine Linse 22 auf Scanner-Spiegel 24, 24' gerichtet und über einen Umlenkspiegel 26 auf das Auge 10 gelenkt wird. Für eine LASIK-Behandlung kann der Laser 20 beispielsweise ein Excimer-Laser sein, dessen Strahlungswellenlänge bei 193 nm liegt. Es versteht sich, dass für andere Behandlungszwecke gewünschtenfalls auch andere Behandlungswellenlängen verwendet werden können. Ein programmgesteuerter Rechner C steuert den Laser 20 und die Scanner-Spiegel 24, 24' gemäß einem zuvor berechneten Behandlungsprofil. Im Folgenden wird davon ausgegangen, dass mit der dargestellten chirurgischen Vorrichtung eine LASIK-Behandlung durchgeführt wird; dementsprechend wird als Behandlungsprofil ein Ablationsprofil angenommen.

Die laserchirurgische Vorrichtung besitzt darüber hinaus eine Einrichtung zur Verfolgung von Augenbewegungen (Eye-Tracker). Der Eye-Tracker umfasst eine Kamera 30, mit der über einen Umlenkspiegel 28 in Richtung eines Pfeils 32 Bilder des Auges, konkret der Pupille und der Iris aufgenommen werden. Die aufgenommenen Bilder werden sodann in dem Rechner C oder in einer nicht näher dargestellten, vorgeschalteten Bildverarbeitungseinheit ausgewertet, um Bewegungen des Auges, die der Patient trotz der versuchten Blickfixierung auf das Fixationslicht 18' in der Regel nicht vermeiden kann, zu verfolgen. Die detektierten Augenbewegungen berücksichtigt der Rechner C bei der Steuerung der Scanner-Spiegel 24, 24', um so das Ablationsprofil möglichst konstant gegenüber einem vorgegebenen Referenzpunkt an der Korneaoberfläche ausgerichtet zu halten.

In die laserchirurgische Vorrichtung ist zudem eine Messeinrichtung 34 für die OLCR (Optical Low Coherence Reflectometry) integriert, die in an sich bekannter Weise eine Quelle für einen Messstrahl enthält, der über einen Umlenkspiegel 42 auf das Auge 10 gerichtet wird. Die Messeinrichtung 34 empfängt vom Auge 10 reflektierte Strahlung über den Umlenkspiegel 42 auf dem gleichen Weg, auf dem die Messstrahlung der Messeinrichtung 34 ausgesendet wird. Dies ist durch einen Doppelpfeil 36 veranschaulicht.

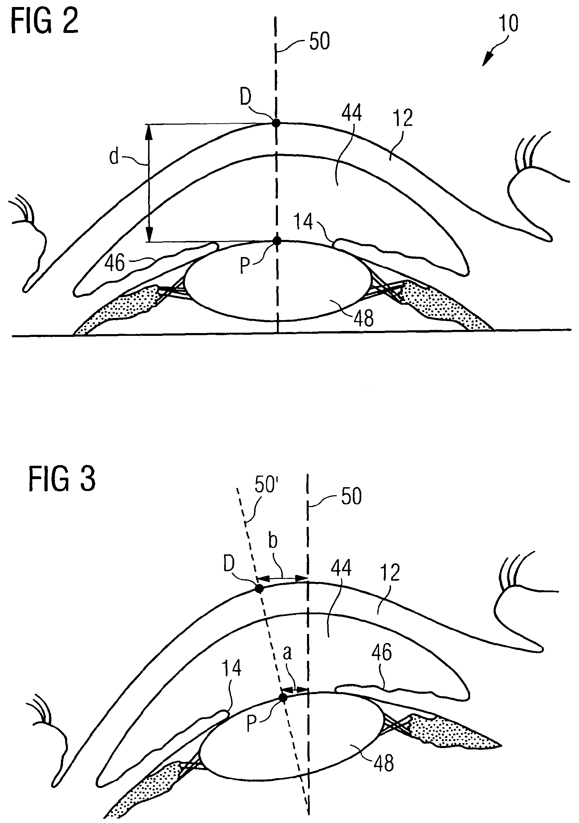

Die Messeinrichtung 34 misst zu Beginn der LASIK, noch bevor der Flap freigeschnitten und weggeklappt wird, die Tiefe der Augenvorderkammer einschließlich der Hornhautdicke. In diesem Zusammenhang wird nun auf Figur 2 verwiesen. Die Augenvorderkammer ist dort mit 44 bezeichnet, 46 bezeichnet die Iris und 48 bezeichnet die Linse des Auges 10. Die Gesamtabmessung von Vorderkammertiefe und Hornhautdicke ist mit d bezeichnet.

In Figur 2 ist ferner eine Pupillenachse 50 eingezeichnet, die einen Pupillenmittelpunkt P mit einem Durchstoßungspunkt D verbindet, an welchem die Pupillenachse 50 die Vorderfläche der Kornea 12 durchstößt.

Eine Augendrehung führt zu einer Verlagerung der Pupillenachse 50 und dementsprechend auch zu einer Verlagerung des Pupillenzentrums P und des Durchstoßungspunkts D. Diese Situation ist in Figur 3 dargestellt. Die neue Pupillenachse ist dort mit 50' bezeichnet. Zum Vergleich ist auch die Pupillenachse 50 des Zustands der Figur 2 eingezeichnet. a und b bezeichnen Wegstrecken, um welche das Pupillenzentrum P bzw. der Durchstoßungspunkt D gegenüber dem Zustand der Figur 2 verschoben sind. Man erkennt, dass der Durchstoßungspunkt D deutlich stärker bei einer Augenbewegung verschoben wird als das Pupillenzentrum P, wobei der Unterschied zwischen den Verschiebemaßen a, b von der Tiefe der Vorderkammer 44 und der Dicke der Hornhaut 12, also insgesamt vom Tiefenmaß d, abhängt.

Der Rechner C der laserchirurgischen Vorrichtung richtet das Ablationsprofil nicht an dem Pupillenzentrum P sondern an dem Durchstoßungspunkt D als Ablationszentrum aus. Hierzu ermittelt er beispielsweise die Lage des Pupillenzentrums P und die Lage des Durchstoßungspunkts D einmal vor Beginn der Operation und merkt sich (speichert) die so ermittelten Werte als Referenzlagen. Während der Laserbehandlung ermittelt der Rechner C fortlaufend anhand der Bilddaten der Kamera 30 die jeweils aktuelle Lage des Pupillenzentrums P und berechnet einen Verschiebungsvektor, der Stärke und Richtung der Verschiebung des Pupillenzentrums P zwischen der gespeicherten Referenzlage und dem aktuellen Zustand angibt. Aus dem so ermittelten Verschiebungsvektor für das Pupillenzentrum P kann sich der Rechner C anhand des gemessenen Tiefenmaßes d und eines ebenfalls messtechnisch gewonnenen oder standardmäßig vorgegebenen Drehradius des Auges 10 einen Verschiebungsvektor für den Durchstoßungspunkt D berechnen. Diese Berechnung ist mit einfacher Mathematik möglich, etwa mit Hilfe des wohlbekannten Strahlensatzes der Geometrie. Aus dem so gewonnenen Verschiebungsvektor für den Durchstoßungspunkt D und der gespeicherten Referenzlage dieses Punkts kann sich der Rechner C anschließend die aktuelle Lage des Durchstoßungspunkts D berechnen. Es ist ohne weiteres ersichtlich, dass der Rechenaufwand für diese Berechnungen vergleichsweise gering ist.

## Patentansprüche

1. Vorrichtung für die ophthalmologische, insbesondere refraktive Laserchirurgie, umfassend
- eine Laserstrahlquelle (20),
- Strahlführungsmittel (24, 24', 26) zum orts- und zeitgesteuerten Führen des von der Laserstrahlquelle emittierten Laserstrahls über ein zu behandelndes Auge,
- eine Kamera (30) zum Aufnehmen eines Bilds der Iris und Pupille des Auges,
- einen mit der Kamera verbundenen, programmgesteuerten Rechner (C) zum Steuern der Strahlführungsmittel nach Maßgabe eines Bearbeitungsprofils, wobei der Rechner dazu eingerichtet ist, während der Behandlung des Auges auf Grundlage der von der Kamera gelieferten Bilddaten die Lage eines vorgegebenen Punkts an der Hornhaut des Auges zu ermitteln und das Bearbeitungsprofil relativ zu der so ermittelten Lage des Hornhautpunkts auszurichten,
- eine ihre Messdaten an den Rechner liefernde Messeinrichtung (34) zur Messung eines für die Vorderkammertiefe und gewünschtenfalls die Hornhautdicke repräsentativen Tiefenmaßes (d) des zu behandelnden Auges,
**dadurch gekennzeichnet, dass** der Rechner (C) dazu eingerichtet ist, aus den Bilddaten der Kamera (34) ein Verschiebungsmaß (a) für das Pupillenzentrum (P) zu ermitteln und die Lage des vorgegebenen Hornhautpunkts abhängig von dem so ermittelten Verschiebungsmaß, dem gemessenen Tiefenmaß (d) und einem
messtechnisch gewonnenen oder standardmäßig vorgegebenen Drehradius des Auges zu ermitteln, wobei das Verschiebungsmaß Richtung und Stärke einer Verschiebung des Pupillenzentrums (P) gegenüber einer Referenzlage des Pupillenzentrums aufgrund einer Rotationsbewegung des Auges repräsentiert.

2. Einrichtung nach Anspruch 1,
**dadurch gekennzeichnet, dass** die Messeinrichtung (34) eine kohärenzoptische interferometrische Messeinrichtung ist, insbesondere eine OLCR-Messeinrichtung.

3. Einrichtung nach einem der vorhergehenden Ansprüche,
**dadurch gekennzeichnet, dass** der vorgegebene Hornhautpunkt der Durchstoßpunkt (D) der Pupillenachse (50) durch die Hornhautoberfläche oder ein mit diesem Durchstoßpunkt in fester relativer Lage stehender Punkt ist.

4. Steuerprogramm für eine Vorrichtung für die ophthalmologische, insbesondere refraktive Laserchirurgie, wobei die Vorrichtung eine Laserstrahlquelle, Strahlführungsmittel zum orts- und zeitgesteuerten Führen des von der Laserstrahlquelle emittierten Laserstrahls über ein zu behandelndes Auge, eine Kamera zum Aufnehmen eines Bilds der Iris und Pupille des Auges und einen mit der Kamera verbundenen, das Steuerprogramm ausführenden Rechner zum Steuern der Strahlführungsmittel nach Maßgabe eines Bearbeitungsprofils umfasst, wobei das Steuerprogramm derart ausgestaltet ist, dass der Rechner während der Behandlung des Auges auf Grundlage der von der Kamera gelieferten Bilddaten die Lage eines vorgegebenen Punkts an der Hornhaut des Auges ermittelt und das Bearbeitungsprofil relativ zu der so ermittelten Lage des Hornhautpunkts ausrichtet,
**dadurch gekennzeichnet, dass** das Steuerprogramm dazu eingerichtet ist, aus den Bilddaten der Kamera (34) ein Verschiebungsmaß (a) für das Pupillenzentrum (P) zu ermitteln und die Lage des vorgegebenen Hornhautpunkts abhängig von dem so ermittelten Verschiebungsmaß, einem für die Vorderkammertiefe und gewünschtenfalls die Hornhautdicke repräsentativen gemessenen Tiefenmaß (d) des zu behandelnden Auges und einem messtechnisch
gewonnenen oder standardmäßig vorgegebenen Drehradius des Auges zu ermitteln, wobei das
Verschiebungsmaß Richtung und Stärke einer Verschiebung des Pupillenzentrums (P) gegenüber einer Referenzlage des Pupillenzentrums aufgrund einer Rotationsbewegung des Auges repräsentiert.

5. Verfahren zum Erzeugen eines Steuerprogramms für einen programmgesteuerten Rechner einer Vorrichtung für die ophthalmologische, insbesondere refraktive Laserchirurgie, wobei die Vorrichtung dazu eingerichtet ist, Laserstrahlung gemäß einer durch ein gewünschtes Bearbeitungsprofil bestimmten, relativ zu einem vorgegebenen Ort eines zu behandelnden Auges ausgerichteten Raum- und Zeitfolge auf oder in das Auge zu richten,
wobei bei dem Verfahren zumindest einmal vor der Chirurgie ein für die Vorderkammertiefe und gewünschtenfalls die Hornhautdicke repräsentatives Tiefenmaß des zu behandelnden Auges gemessen wird und das Steuerprogramm so erzeugt wird, dass es während der Chirurgie aus Bilddaten einer Kamera ein Verschiebungsmaß (a) für das Pupillenzentrum (P) ermittelt und den vorgegebenen Ort abhängig von dem so ermittelten Verschiebungsmaß, dem gemessenen Tiefenmaß (d) und einem messtechnisch gewonnenen oder standardmäßig vorgegebenen Drehradius des Auges ermittelt, wobei das Verschiebungsmaß Richtung und Stärke einer Verschiebung des Pupillenzentrums (P) gegenüber einer Referenzlage des Pupillenzentrums aufgrund einer Rotationsbewegung des Auges repräsentiert.

## Claims

1. Apparatus for ophthalmological, in particular, refractive laser surgery, comprising
- a laser-beam source (20),
- beam guidance means (24, 24', 26) for location- and time-controlled guidance of the laser beam, emitted by the laser-beam source, over an eye to be treated,
- a camera (30) for taking an image of the iris and pupil of the eye,
- a program-controlled computer (C), connected to the camera, for controlling the beam guidance means in accordance with a treatment profile, the computer being configured to ascertain during the treatment of the eye, on the basis of the image data supplied by the camera, the position of a specified point on the cornea of the eye and to align the treatment profile relative to the thus ascertained position of the corneal point,
- a measuring device (34), supplying its measurement data to the computer, for measuring a depth dimension (d) of the eye to be treated, which depth dimension is representative of the depth of the anterior chamber and, if desired, of the thickness of the cornea,
**characterized in that** the computer (C) is configured to ascertain a displacement dimension (a) for the pupil center (P) from the image data of the camera (34) and to ascertain the position of the specified corneal point based on the so-ascertained displacement dimension, the measured depth dimension (d) and a radius of rotation of the eye, which has been obtained by way of measurement or is pre-defined by way of default, wherein the displacement dimension represents the direction and amount of a displacement of the pupil center (P) with respect to a reference position of the pupil center due to a rotational movement of the eye.

2. Apparatus according to Claim 1,
**characterized in that** the measuring device (34) is a coherent optical interferometric measuring device, in particular an OLCR measuring device.

3. Apparatus according to any one preceding claim,
**characterized in that** the specified corneal point is the point (D) at which the pupil axis (50) pierces through the surface of the cornea, or is a point that is in a fixed relative position in relation to this piercing point.

4. Control program for an apparatus for ophthalmological, in particular, refractive laser surgery, the apparatus comprising a laser-beam source, beam guidance means for location- and time-controlled guidance of the laser beam, emitted by the laser-beam source, over an eye to be treated, a camera for taking an image of the iris and pupil of the eye, and a computer, which is connected to the camera and executes the control program, for controlling the beam guidance means in accordance with a treatment profile, the control program being designed in such a way that the computer ascertains during the treatment of the eye, on the basis of the image data supplied by the camera, the position of a specified point on the cornea of the eye and aligns the treatment profile relative to the thus ascertained position of the corneal point, **characterized in that** the control program is designed to ascertain a displacement dimension (a) for the pupil center (P) from the image data of the camera (34) and ascertain the position of the specified corneal point based on the so-ascertained displacement dimension, a measured depth dimension (d) of the eye to be treated, which depth dimension is representative of the depth of the anterior chamber and, if desired, of the thickness of the cornea, and a radius of rotation of the eye, which has been obtained by way of measurement or is pre-defined by default, wherein the displacement dimension represents the direction and amount of a displacement of a pupil center (P) with respect to a reference position of the pupil center due to a rotational movement of the eye.

5. Method for generating a control program for a program-controlled computer of an apparatus for ophthalmological, in particular, refractive laser surgery, the apparatus being configured to direct laser radiation onto or into the eye according to a spatial and time sequence that is determined by a desired treatment profile and that is aligned relative to a specified location of an eye to be treated, wherein in the method, a depth dimension of the eye to be treated is measured at least once prior to the surgery, which depth dimension is representative of the depth of the anterior chamber and, if desired, of the thickness of the cornea, and the control program is so generated that, during the surgery, it ascertains a displacement dimension (a) for the pupil center (P) from image data of a camera (34) and ascertains the specified location based on the so-ascertained displacement dimension, the measured depth dimension (d) and a radius of rotation of the eye, which has been obtained by measurement or is pre-defined by default, wherein the displacement dimension represents the direction and amount of a displacement of a pupil center (P) with respect to a reference position of the pupil center due to a rotational movement of the eye.

## Revendications

1. Dispositif de chirurgie laser ophtalmologique, en particulier réfractive, comprenant
- une source de faisceau laser (20),
- des moyens de guidage de faisceau (24, 24', 26) pour guider dans l'espace et dans le temps le faisceau laser émis par ladite source de faisceau laser au-dessus d'un oeil à traiter,
- une caméra (30) pour enregistrer une image de l'iris et de la pupille de l'oeil,
- un calculateur (C) commandé par programme et relié à ladite caméra pour commander lesdits moyens de guidage de faisceau en fonction d'un profil de traitement à réaliser, le calculateur (C) étant conçu pour déterminer à partir des données image fournies par la caméra la position d'un point donné sur la cornée de l'oeil pendant l'intervention ophtalmologique et pour aligner le profil de traitement par rapport à la position dudit point ainsi déterminée,
- un dispositif de mesure (34) qui transmet ses données de mesure audit calculateur et sert à mesurer une cote de profondeur (d) représentative de la profondeur de la chambre antérieure et éventuellement de la cornée de l'oeil à traiter,
**caractérisé en ce que** le calculateur (C) est conçu pour déterminer à partir des données image fournies par la caméra (34) une cote de décalage (a) pour le centre (P) de la pupille et pour déterminer la position dudit point sur la cornée en fonction de la cote de décalage ainsi déterminée, en fonction de la cote de profondeur (d) mesurée et en fonction d'un rayon de rotation de l'oeil défini par défaut ou par mesure, cette cote de décalage représentant la direction et l'intensité d'un décalage du centre (P) de la pupille par rapport à une position de référence du centre de la pupille, suite à un mouvement de rotation de l'oeil.

2. Dispositif selon la revendication 1,
**caractérisé en ce que** ledit dispositif de mesure (34) consiste en un dispositif de mesure par interférométrie optique cohérente, plus particulièrement en un dispositif de mesure OLCR.

3. Dispositif selon l'une des revendications précédentes,
**caractérisé en ce que** le point donné sur la cornée est le point d'intersection (D) de l'axe pupillaire (50) avec la surface de la cornée, ou un point en position fixe par rapport à ce point d'intersection.

4. Programme de commande pour dispositif de chirurgie laser ophtalmologique, en particulier réfractive, ce dispositif comprenant une source de faisceau laser (20), des moyens de guidage de faisceau (24, 24', 26) pour guider dans l'espace et dans le temps le faisceau laser émis par la source de faisceau laser au-dessus d'un oeil à traiter, une caméra (30) pour enregistrer une image de l'iris et de la pupille de l'oeil, un calculateur (C) relié à cette caméra et exécutant le programme commande pour commander les moyens de guidage de faisceau en fonction d'un profil de traitement, le programme de commande étant conçu de sorte que le calculateur détermine à partir des données image fournies par la caméra la position d'un point donné sur la cornée de l'oeil pendant l'intervention ophtalmologique et aligne le profil de traitement par rapport à la position du point sur la cornée ainsi déterminée,
**caractérisé en ce que** le programme de commande est conçu pour déterminer à partir des données fournies par la caméra (34) une cote de décalage (a) pour le centre (P) de la pupille et pour déterminer la position du point donné sur la cornée en fonction de la cote de décalage ainsi déterminée, en fonction de la cote de profondeur (d) mesurée et représentative de la profondeur de la chambre antérieure et éventuellement de l'épaisseur de la cornée pour l'oeil à traiter et en fonction d'un rayon de rotation de l'oeil donné par défaut ou obtenu par mesure, la cote de décalage représentant la direction et l'intensité d'un décalage du centre (P) de la pupille par rapport une position de référence du centre de la pupille, suite à un mouvement de rotation de l'oeil.

5. Procédé pour générer un programme de commande pour calculateur commandé par programme d'un dispositif de chirurgie laser ophtalmologique, en particulier réfractive, ce dispositif étant conçu pour diriger sur ou dans l'oeil le faisceau laser selon une séquence spatiale et temporelle déterminée par un profil de traitement souhaité et définie par rapport à un endroit donné de l'oeil à traiter,
dans le cadre duquel procédé une cote de profondeur représentative de la profondeur de la chambre antérieure et éventuellement de l'épaisseur de la cornée de l'oeil à traiter étant mesurée au moins une fois avant l'intervention chirurgicale et le programme de commande étant généré de manière à calculer pendant l'intervention chirurgicale et à partir des données image fournies par une caméra une cote de décalage (a) pour le centre (P) de la pupille et à déterminer l'endroit donné en fonction de la cote de décalage ainsi calculée, en fonction de la cote de profondeur (d) mesurée et en fonction d'un rayon de rotation de l'oeil défini par défaut ou défini par mesure, cette cote de décalage représentant la direction et l'intensité d'un décalage du centre (P) de la pupille par rapport une position de référence du centre de la pupille, suite à un mouvement de rotation de l'oeil.
